(19) Europäisches Patentamt
European Patent Office
Office européen des brevets

(11) Publication number: **0 034 480**
**A2**

(12) # EUROPEAN PATENT APPLICATION

(21) Application number: 81300598.0

(22) Date of filing: 13.02.81

(51) Int. Cl.³: C 07 C 85/06
C 07 D 295/02

(30) Priority: 14.02.80 GB 8004984

(43) Date of publication of application: 26.08.81 Bulletin 81 34

(84) Designated Contracting States: AT BE CH DE FR GB IT LI LU NL SE

(71) Applicant: Grigg, Ronald Ernest
The Queen's University of Belfast Department of Chemistry David Keir Building
Belfast Northern Ireland(GB)

(72) Inventor: Grigg, Ronald Ernest The Queen's University of Belfast Dept of Chemistry David Keir Building
Belfast Northern Ireland(GB)

(72) Inventor: Sutthivaiyakit, Somyote Chemistry Dept
Faculty of Science Ramkamhaeng
Huamark, Bangkok(TH)

(74) Representative: Perry, Robert Edward et al,
GILL JENNINGS & EVERY 53-64 Chancery Lane
London WC2A 1HN(GB)

(54) **Alkylation of amines.**

(57) A process for preparing a N-alkylamine or N, N-dialkylamine, which comprises reacting a primary or secondary amine with a primary or secondary alcohol in the presence of a catalyst selected from iridium, rhodium, ruthenium, osmium, platinum, palladium and rhenium, present either as the metal or a salt or complex thereof. The catalyst is preferably a complex such as $RhH(PPh_3)_4$. The process can be used to cause cyclisation, including internal cyclisation so that, by way of example, N-methyl-pyrrolidine may be prepared from (1) pyrrolidine and methanol; (2) 1,4-butanediol and methylamine; and (3) 4-methylamino-1-butanol.

EP 0 034 480 A2

# ALKYLATION OF AMINES

This invention relates to a process for the alkylation of amines, by which N-alkylamines and/or N,N-dialkylamines can be obtained from primary and secondary amines.

One known method for alkylating a primary or secondary amine involves the use of an alkyl halide as the alkylating agent. This process suffers from the disadvantage that mixtures, including tetraalkylammonium salts, are produced. Another known amine alkylation process comprises reductive alkylation, using a carbonyl compound to form an intermediate imine which is then reduced. This second process often requires forcing conditions and/or a separate step for the reduction.

It is well known to use noble metal compounds salts or complexes as catalysts in reactions in the organic chemistry field. For example, it is well known to use platinum-on-carbon or palladium-on-carbon as a hydrogenation catalyst, and rhodium catalysts have been used, e.g. for converting a mixture of carbon monoxide and hydrogen to methanol. The complex $RhH(PPh_3)_4$ is disclosed as a hydrosilylation catalyst in Chem. Abs. 1976, 85, 124132u.

It has now been found that noble metal catalysts can be used to give surprisingly good yields in the alkylation of amines. Further, such reactions have been found to provide, in certain circumstances, selective monoalkylation and even selective monoalkylation of the primary amine in a compound containing both a primary amino group and a secondary amino group.

According to the present invention, a N-alkylamine or N,N-dialkylamine is prepared by reacting a primary or secondary amine with a primary or secondary alcohol in the presence of a catalyst selected from iridium, rhodium, ruthenium, osmium, platinum, palladium and rhenium, present either as the metal or a salt or complex thereof. Any catalyst which is present as metal is preferably present as metal supported on an appropriate support, such as carbon/ or a suitable polymer. Salts or complexes can likewise be carried on a support, but this generally unnecessary. Any supported catalyst is preferably in particulate form.

Suitable catalysts in which the catalytic material is present as metal include palladium or rhodium supported in charcoal. However, the preferred form of the catalyst is as a salt or complex of the catalytic metal. In order to increase their stability during the reaction, and potentiate the catalytic activity, salts are themselves preferably stabilised, for example, by the addition of triphenylphosphine / or chelating diphosphines. The salts may be formed with salt-forming radicals such as chloride but others

which may be used include bromide and acetate. Preferred catalysts for use in the invention are the complexed salts of rhodium and iridium, for example, the complexes formed between iridum chloride ($IrCl_3H_2O$) and triphenylphosphine or other stabilising ligand. The molar ratio of salt to stabilising ligand may be, for example, 1:3, 1:4 or 1:5. Other useful salts include $Na_2IrCl_6$, $RhCl_3.3H_2O$, $RuCl_2(PPh_3)_3$ and $RuCl_3.H_2O$. However, the most préferred catalysts are complexed hydrides of the catalytic metal, e.g. $RuH_2(PPh_3)_4$, mer-$IrH_3(PPh_3)_3$ and, especially, $RhH(PPh_3)_4$.

The amine which is alkylated in the process of the invention may be heterocyclic, aliphatic or aromatic. Examples of suitable aromatic amines are aniline and toluidines, but these are often less preferred.

Heterocyclic amines which can be alkylated in accordance with the invention may be saturated or unsaturated, and may thus be aromatic, and the rings may contain hetero atoms such as N, O or S in addition to the nitrogen which is to be alkylated. Such a ring generally has 5 or 6 members. Suitable heterocylcic amines include morpholine, piperidine, pyrrolidine and piperazine. Alkylation of piperazine or any other amine having two primary or secondary amino groups can result in alkylation occuring at both amino nitrogen atoms. The heterocyclic amine can be substituted anywhere in the ring by any substituents which do not interfere with the alkylation.

Aliphatic amines may be cyclic or linear. Cyclic aliphatic amines may contain a hydrocarbon ring of 5 to 8,

usually 5 or 6, carbon atoms, an example being cyclohexylamine. Aliphatic amines may be saturated or unsaturated. They may be primary or secondary and, if secondary, the substituents on the nitrogen atom may be the same or different. Linear aliphatic amines include diallylamine, butylamine and N-methylbutylamine.

The molecular weight of the amine used in the invention is not critical; it may contain up to 18, or more, carbon atoms. The alcohol used in the invention may contain, for instance up to 18, e.g. 1 to 10, and preferably 1 to 4, carbon atoms. The alcohol is usually a primary alcohol. It is often preferably an alkanol such as methanol, ethanol or isopropanol. The use of methanol, for N-methylation, in the invention is very convenient. However, the alcohol may also be, for example, an alkanol carrying one or more non-interfering substituents or an aralkyl alcohol such as benzyl alcohol.

The process of the invention can be used to cause cyclisation and the formation of a heterocyclic ring containing the amine N atom, (1) if a primary amine is reacted with a diol, or (2), causing internal cyclisation, by using a difunctional compound containing both a primary or secondary amino group and a primary or secondary alcohol group. Clearly, the diol or difunctional compound should contain a number of atoms in the chain between its reactive groups which will allow cyclisation to occur and this number should be at least 4 and is usually 4, 5 or, possibly, 6. Examples of suitable diols are 1,4-butanediol, 1,5-pentanediol and 2-phenyl-1,4-pentanediol. 1,4-Butanediol can be reacted with benzylamine to give N-benzylpyrrolidine; 1,5-pentanediol and methylamine give N-methylpiperidine.

General examples of compounds which can be internally cyclised are alkylamines having a 4- or 5-hydroxyl group; the amine may be a primary or secondary amine or may form part of a heterocyclic ring. Specific examples of compounds of this type are 4-amino-1-butanol (which gives pyrrolidine on cyclisation), 4-(N-n-butyl-

amino)-1-butanol, 4-(N-benzylamino)-1-butanol, 5-amino-1-pentanol, 6-amino-3-hexanol, 2-(3-hydroxypropyl)piperidine and 2-(4-hydroxybutyl)piperidine. The products which are obtained by the use of the last two of these specific compounds in the process of the invention are, respectively, 1-azabicyclo[4.3.0]nonane and 1-azabicyclo[4.4.0]decane.

The process of the invention is preferably conducted by reacting the alcohol and amine in the presence of the catalyst in a solvent at the chosen reaction temperature. The solvent may be any inert organic solvent, i.e. one that does not interfere with the reaction, but preferably it is provided by the alcohol, which is therefore preferably present in excess. Ethereal solvents such as dioxan may be preferred, although hydrocarbons such as benzene can also be used.

The amount of catalyst based on the amine may be, for instance, 1 to 10 mole %, preferably about 5 mole %. The reaction is preferably conducted at the boiling point of the alcohol and most conveniently under reflux at atmospheric pressure.

The duration of reaction may vary but is often lower than for known alkylation reactions for the production of parallel compounds. As indicated above, the process of the invention can give selective monoalkylation and it appears that, when alkylating a primary amine, the alkylation proceeds to give first the monoalkylated and then the dialkylated product. Therefore, if the monoalkylated product is desired, the reaction can be stopped at a point before any substantial degree of dialkylation has occurred. Again, because alkylation of secondary amines generally takes longer than monoalkylation of primary amines, a compound such as spermine can be alkylated by the process of the present invention to give, say,

substantially only N,N'-bis(3-methylaminopropyl)-1,4-butanediamine.

Generally, the duration of the reaction will be from 1 to 72 hours but will often not need to be longer than 10 or even 5 hours for the monoalkylation of primary amines.

In the alkylation reaction to which the present invention relates, the catalyst may be recovered by conventional means. A supported catalyst may be recovered most easily.

It appears that the alcohol is initially oxidised to the corresponding aldehyde, forming a metal hydride catalyst; the aldehyde then reacts with the amine to form an imine which is then reduced by the metal hydride to form the amine, thereby releasing the initial metal catalyst. The reaction can be illustrated as follows, wherein M represents the metal catalyst, $QNH_2$ represents the amine to be alkylated, and R represents hydrogen, alkyl or aryl:

$$M + RCH_2OH \text{ —— } MH_2 + RCHO$$

$$QNH_2 + RCHO \text{ —— } QNH{=}CHR + H_2O$$

$$QNH{=}CHR + MH_2 \text{ —— } QNHCH_2R + M$$

The following Examples illustrate the invention. All percentages are by weight, yields of N-alkylated products being expressed with respect to the starting amine prior to alkylation.

Examples 1 to 6 N-Methylpyrrolidine

Pyrrolidine was reacted with 5 mole % of six different catalysts under reflux in methanol at 64°C, using a bath at 100°C. The reaction mixture was then worked up to yield the title product. The catalysts,

reaction times and yields are given in Table I (in Example 1, the conversion was 90.6%).

TABLE I

| Example | Catalyst | Time (h) | Yield (%) |
|---|---|---|---|
| 1 | $IrCl_3.H_2O(PPh_3)_5$ | 22 | 78.9 |
| 2 | $PhCl(PPh_3)_3$ | 5 | 66.4 |
| | | 8 | 92.4 |
| 3 | $RhH(PPh_3)_4$ | 4 | 97.2 |
| 4 | $IrCl(PPh_3)_3$ | 5 | 87.3 |
| 5 | mer-$IrH_3(PPh_3)_3$ | 24 | 46.9 |
| 6 | $RuH_2(PPh_3)_4$ | 48 | 15.0 |

The process of Example 1 was repeated at 78°C with ethanol, at 82°C with isopropanol and at 100°C with benzyl alcohol. N-ethylpyrrolidine, N-isopropylpyrrolidine and N-benzylpyrrolidine, respectively, were obtained in satisfactory yield. The process was also repeated replacing the pyrrolidine with equimolar amounts of benzylamine, morpholine, piperazine, cyclohexylamine, diallylamine, 2,6-dimethylpiperidine and 3,4-dimethylpiperidine, and in all instances the appropriate alkylated derivative of the amine was obtained.

Analogously, piperidine can be reacted with methanol under reflux at 64°C using other catalysts, namely $IrCl_3H_2O$ and $RhCl_3.3H_2O$. Although satisfactory yields in many instances could be obtained with reflux times as short as 9 hours, optimum yields in some instances required longer reflux times, e.g. up to 240 hours.

<u>Example 7</u> N-Methylbutylamine

A mixture of n-butylamine (0.66 g, 9.04 x $10^{-3}$ mole), $RhH(PPh_3)_4$ (0.53 g, 4.6 x $10^{-4}$ mole), mesitylene (0.189 g, 1.57 x $10^{-3}$ mole, as internal standard) in methanol (6.5 ml) was heated under reflux and magnetically stirred in an oil bath under a small positive pressure of nitrogen. The progression of the reaction was monitored by glc (4 m, 5% KOH + 20% Carbowax 20 M on Chromosorb W, $50^o$). Glc showed 84% yield of the monoalkylated title product after three hours and 98.5% yield after 8 hours. Dimethylation of the butylamine was observed after a reaction time greater than 24 hours.

<u>Example 8</u> N-Methylcyclohexylamine

A mixture of cyclohexylamine (0.7 g, 7.07 x $10^{-3}$ mole), $RhH(PPh_3)_4$ (0.41 g, 3.56 x $10^{-4}$ mole), mesitylene (0.15 g, 1.25 x $10^{-3}$ mole, as internal standard) in methanol (5 ml) was heated under reflux and magnetically stirred in an oil bath under a small positive pressure of nitrogen. Glc (4 m, 5% SGR, $140^o$) was used to monitor the progression of the reaction, to identify the product and to evaluate the yields. The monomethylated product was obtained in 81.9% yield after 4 hours and 98.9% after 10 hours. Dialkylation was observed after more than 24 hours.

When the procedure of Example 8 was repeated using 0.8 g of the catalyst (1 mole % with respect to cyclohexylamine) the yields after 4 and 22 hours were 56.8% and 88.2%, respectively.

<u>Example 9</u> N,N-Dimethylbutylamine

A mixture of N-methyl-n-butylamine (0.79 g,

$9.08 \times 10^{-3}$ mole), $RhH(PPh_3)_4$ (0.53 g, $4.6 \times 10^{-4}$ mole), mesitylene (0.189 g, $1.57 \times 10^{-3}$ mole, as internal standard) in methanol (6.5 ml) was heated under reflux and magnetically stirred in an oil bath under a small positive pressure of nitrogen. The progression of the reaction was monitored by glc (4 m, 5% KOH + 20% Carbowax 20 M on Chromosorb W, $50^{o}$). Glc showed 45%, 57.3% and 86% yields of the title product after 10 hours, 24 hours and 4 days, respectively. The catalyst was then filtered from the cooled reaction mixture using a sintered glass flunnel. The filtrate was fractionated at atmospheric pressure.

Example 10 N-Benzylpyrrolidine

A mixture of pyrrolidine (0.75 g, $1.06 \times 10^{-2}$ mole), $RhH(PPh_3)_4$ (0.165 g, $5.34 \times 10^{-4}$ mole), mesitylene (0.226 g, $1.88 \times 10^{-3}$ mole, as internal standard) in benzyl alcohol (7.5 ml) was heated under reflux and magnetically stirred in an oil bath under a small positive pressure of nitrogen. The progression of the reaction was monitored by glc (2 m, Carbowax 20 M, 60 - $200^{o}$). The title product was obtained in 99% yield (by glc) after 4 hours.

Example 11 N-Methylaniline

A mixture of aniline (0.34 g, $3.65 \times 10^{-3}$ mole), $RhH(PPh_3)_4$ (0.213 g, $1.85 \times 10^{-4}$ mole), mesitylene (0.099 g, $8.25 \times 10^{-4}$ mole, as internal standard) in methanol (2.7 ml) was heated under reflux and magnetically stirred in an oil bath under a small positive pressure of nitrogen. The progression of the reaction was monitored by glc (6 m, 15% Carbowax, $175^{o}$). After 1, 2 and 3 days,

the respective yields observed were 16.2%, 26.6% and 38.7%.

When Example 11 was repeated using the same amount of catalyst in admixture with an equimolar amount of sodium carbonate, the yields after 1 and 2 days were 6.18% and 11.4%, respectively.

Example 12 N-n-Butylpyrrolidine (by cyclisation)

A mixture of 4-(N-n-butylamino)-1-butanol (1.5 g, 1.03 x $10^{-2}$ mole), $RhH(PPh_3)_4$ (0.596 g, 5.17 x $10^{-4}$ mole) in dioxan (19 ml) was heated under reflux and magnetically stirred in an oil bath under a small positive pressure of nitrogen. The progression of the reaction was monitored by glc (2 m, Carbowax 20 M, 60 - 170$^o$) following the addition of chloroform and several washes with water to remove dioxane. After 5 hours, the reaction mixture was filtered through a sintered glass funnel. Chloroform (200 ml) was added to the filtrate and the mixture was washed with water several times to remove dioxan. The organic layer was dried with anhydrous $Na_2SO_4$, the chloroform removed and the residual liquid fractionated to give the title product (0.73 g, 56%) and the unreacted starting material (0.39 g).

When the procedure of Example 12 was repeated using benzene as solvent, the yield after 24 hours was 4.98%.

Example 13 N-Benzylpyrrolidine (by cyclisation)

The procedure of Example 12 was repeated using. instead of the 4-(N-butylamino)-1-butanol, an equimolar amount of 4-(N-benzylamino)-1-butanol. The title product was obtained in 49.3% yield after 5 hours and in 82.6% yield after 24 hours, as determined by glc.

Example 14 N-Benzylpyrrolidine (by cyclisation)

The procedure of Example 12 was repeated, using instead of the 4-(N-butylamino)-1-butanol, an equimolar amount of benzylamine and twice as much, on a molar basis, of 1,4-butanediol. The title product was obtained in 8.7% yield after 7 days. A yield of 31.35% of the title product was obtained after 5 days when the molar ratio of the diol to the amine was 10:1 instead of 2:1.

It will be appreciated that the process of the present invention can provide a variety of products in good yields. For example, N-methylpyrrolidine can be prepared by the process of the invention in three ways; (1) from pyrrolidine and methanol; (2) from 1,4-butanediol and methylamine; and (3) from 4-methylamino-1-butanol.

CLAIMS

1. A process for preparing a N-alkylamine or N,N-dialkylamine, which comprises reacting a primary or secondary amine with a primary or secondary alcohol in the presence of a catalyst selected from iridium, rhodium, ruthenium, osmium, platinum, palladium and rhenium, present either as the metal or a salt or complex thereof.

2. A process according to claim 1 in which the catalyst is iridium or rhodium, or a salt or complex thereof.

3. A process according to claim 1 or claim 2 in which the catalyst is a complex, preferably the metal hydride complex, and most preferably $RhH(PPh_3)_4$.

4. A process according to any preceding claim in which the amine is a heterocyclic or aliphatic, and preferably an aliphatic, amine.

5. A process according to any preceding claim in which the alcohol is a primary alcohol,and preferably an alkanol.

6. A process according to any preceding claim in which the amine is a primary amine and the product is the monoalkylated amine.

7. A process according to any preceding claim in which the amine and the alcohol group are present in the same compound in which they are separated by a chain of at least 4 atoms.